# EUROPEAN PATENT APPLICATION

(11) **EP 4 379 375 A1**
(43) Date of publication of application: **05.06.2024**
(21) Application number: 23213200.1
(22) Date of filing: 30.11.2023
(51) Int. Cl.: G01N 33/50, G01N 33/569, G01N 33/68

(54) **CLASSIFICATION OF CONNECTIVE TISSUE DISEASE WITH NEUROLOGICAL MANIFESTATION USING MULTI-DIMENSIONAL PERIPHERAL BLOOD ANALYSIS**

(30) Priority: 01.12.2022 EP 22210716
(71) Applicant: Universität Münster, 48149 Münster (DE)
(72) Inventor: HEMING, Michael, 48149 Münster (DE); MEYER ZU HOERSTE, Gerd, 48149 Münster (DE); WIENDL, Heinz, 48149 Münster (DE)
(74) Representative: Schiweck Weinzierl Koch Patentanwälte Partnerschaft mbB

(57) **Abstract**

The present invention relates to a method of diagnosing a subject with connective tissue disease with neurological manifestation comprising determining certain cell levels and ratios of cell levels in a test peripheral blood sample obtained from a subject suffering from rheumatic symptoms and diagnosing said subject as suffering from said disease if certain criteria are fulfilled relative to the corresponding cell levels and ratios of cell levels in control peripheral blood samples obtained from subjects suffering from connective tissue disease without neurological manifestation. Further, the present invention relates to a data processing system comprising a processor configured to perform the method of the invention, a flow cytometry device capable of detecting the specific cell levels, a computer program comprising instructions to cause the data processing system or the flow cytometry device to execute the steps of the method of the invention and a computer-readable medium having stored thereon the computer program.

## Description

### TECHNICAL FIELD OF THE INVENTION

The present invention relates to a method of diagnosing a subject with connective tissue disease with neurological manifestation comprising determining certain cell levels and ratios of cell levels in a test peripheral blood sample obtained from a subject suffering from rheumatic symptoms and diagnosing said subject as suffering from said disease if certain criteria are fulfilled relative to the corresponding cell levels and ratios of cell levels in control peripheral blood samples obtained from subjects suffering from connective tissue disease without neurological manifestation. Further, the present invention relates to a data processing system comprising a processor configured to perform the method of the invention, a flow cytometry device capable of detecting the specific cell levels, a computer program comprising instructions to cause the data processing system or the flow cytometry device to execute the steps of the method of the invention and a computer-readable medium having stored thereon the computer program.

### BACKGROUND OF THE INVENTION

Connective tissues are made up of two proteins: collagen and elastin. Collagen is a protein found in the tendons, ligaments, skin, cornea, cartilage, bone and blood vessels. Elastin is a stretchy protein that resembles a rubber band and is the major component of ligaments and skin. When a subject has a connective tissue disease, the collagen and elastin are inflamed. The proteins and the body parts they connect are harmed.

Three of the most frequent connective tissue diseases and vasculitides, which can be summarized as connective tissue diseases (CTDs) for the sake of simplicity, are systemic lupus erythematodes (SLE), granulomatosis with polyangiitis (GPA), and primary Sjörgen's syndrome (SS). Although their exact triggers are still a subject of debate, both genetic and microbiota/environmental factors likely contribute (Ruff et al. 2020; Nat Rev Microbiol;18:521-38). Genome-wide association studies and smaller genetic studies have associated disease risk with polymorphisms mainly in the HLA and in other immune-related genetic regions (Harley et al. 2008; Nat Genet 2008;40:204-10 and Kang et al. 1993; J Immunol 1993;150:3615-23) in accordance with the autoimmune etiology of CTD. Neurological manifestations can occur in CTD patients and generally indicate severe tissue inflammation and an unfavorable prognosis (Schwartz et al. 2019; Nat Rev Rheumatol 2019;15:137-52 and Govoni et al. 2001; CNS Drugs 2001;15:597-607). Neurological sequelae in SLE are common but with a highly varying prevalence ranging between 20-50% and include seizures, cognitive dysfunction, headaches, stroke, psychosis, and aseptic meningitis (Govoni and Hanly 2020; Rheumatology 2020;59:v52-62). Neurological involvement in SS is also common and peripheral neuropathy is likely its most common complication (2-10%) (Brito-Zerón et al. 2013; Clin Exp Rheumatol 31 :103-10). GPA patients develop neurological manifestations (-30%), especially due to peripheral and cranial neuropathy (Nishino et al. 1993; Annals of Neurology 1993;33:4-9). Differentiating CTD without neurological manifestation from CTD patients with neurological manifestations (henceforth referred to as connective tissue disease with neurological manifestation (N-CTD)) is often challenging, but important to guide treatment decisions because patients with N-CTD show an increased mortality (Mak et al. 2012; Semin Arthritis Rheum 2012;41 :830-9) and lower quality of life. In fact, early diagnosis of N-CTDs can improve treatment responses (Papachristos et al. 2021; Semin Arthritis Rheum 2021;51 :49-71). MRI scans, blood samples, electrophysiology, and EEGs can but do not necessarily show abnormalities and if they do, they are non-specific for N-CTD. This makes differentiating N-CTD from CTD difficult.

Therefore, there is a need in the art to provide new methods comprising diagnostic and prognostic surrogates of N-CTD for diagnosing a subject with N-CTD.

The solution of the present invention is described in the following, exemplified in the appended examples, illustrated in the figures and reflected in the claims.

### SUMMARY OF THE INVENTION

The present invention deals with the *in vitro* diagnosis of N-CTD by using a peripheral blood (PB) sample obtained from a subject suffering from rheumatic symptoms and determining certain cell levels in said sample which then enable to diagnose said subject with N-CTD, if particular requirements (criteria) concerning said determined cell levels (parameters) are fulfilled relative to the corresponding cell levels in control PB samples obtained from subjects suffering from CTD. In detail, the inventors found out that a CD4⁺/CD8⁺ ratio and a CD4⁺ T cell level are increased and that the level of classical monocytes are decreased in the blood of N-CTD patients which combination refers to the particular requirements to be fulfilled for diagnosing a subject with N-CTD.

Contrary to other cell parameters in the CSF, which have also been determined by the inventors and turned out to be non-reliable parameters since they have not been significant between N-CTD and CTD patients, the determination of the particularly abovementioned parameters and their characteristics (increase/decrease) compared to corresponding control samples as the particular requirements of the method of the invention are restricted to PB samples only. Thus, the invention reveals that blood, namely PB, alone, in particular that the determination of said specific parameters in the PB, surprisingly suffices to distinguish N-CTD from clinically similar entities such as CTD, suggesting that a rapid blood test could support clinicians in the differential diagnosis of N-CTD based on the particular method of the invention.

Thus, in a first aspect the present invention comprises a method of diagnosing a subject with connective tissue disease with neurological manifestation (N-CTD), comprising a) determining i) a level of CD4⁺ T cells; ii) a level of CD8⁺ T cells; and iii) a level of CD14⁺CD16⁻ monocytes in a test PB sample obtained from a subject suffering from rheumatic symptoms, and b) determining the ratio of the determined level of CD4⁺ T cells of step ai) to the determined level of CD8⁺ T cells of step aii); c) diagnosing said subject as suffering from N-CTD, if the following are fulfilled: i) the ratio of the level of CD4⁺ T cells to the level of CD8⁺ T cells of step b) is increased relative to corresponding ratios of the level of CD4⁺ T cells to the level of CD8⁺ T cells in control PB samples obtained from subjects suffering from CTD; ii) the level of CD4⁺ T cells is increased relative to corresponding levels of CD4⁺ T cells in said control PB samples obtained from said subjects suffering from CTD; and iii) the level of CD14⁺CD16⁻ monocytes is decreased relative to corresponding levels of CD14⁺CD16⁻ monocytes in said control PB samples obtained from said subjects suffering from CTD, wherein the combination of i) - iii) of step c) is indicative for whether said subject already suffering from rheumatic symptoms suffers from N-CTD.

In a second aspect, the present invention envisages a data processing system comprising a processor configured to perform a method comprising the steps of a) obtaining i) the detected level of CD4⁺ T cells; ii) the detected level of CD8⁺ cells; and iii) the detected level of CD14⁺CD16⁻ monocytes, from a test PB sample obtained from a subject suffering from rheumatic symptoms and determining the ratio of the detected level of CD4⁺ T cells of step ai) to the detected level of CD8⁺ T cells of step aii); b) diagnosing said subject as suffering from N-CTD, if the following are fulfilled: i) the determined ratio of the detected level of CD4⁺ T cells to the detected level of CD8⁺ T cells is increased relative to corresponding ratios of the level of CD4⁺ T cells to the level of CD8⁺ T cells in control PB samples obtained from subjects suffering from CTD; ii) the detected level of CD4⁺ T cells is increased relative to corresponding levels of CD4⁺ T cells in said control PB samples obtained from said subjects suffering from CTD; and iii) the detected level of CD14⁺CD16⁻ monocytes is decreased relative to corresponding levels of CD14⁺CD16⁻ monocytes in said control PB samples obtained from said subjects suffering from CTD, wherein the combination of i) - iii) of step b) is indicative for whether said subject already suffering from rheumatic symptoms suffers from N-CTD.

### BRIEF DESCRIPTION OF THE FIGURES

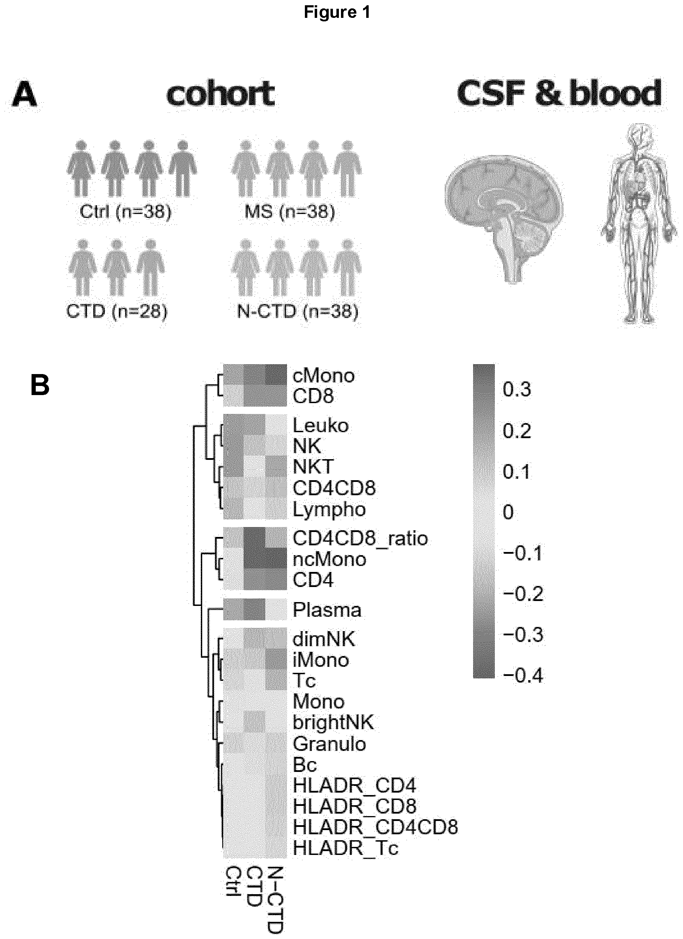

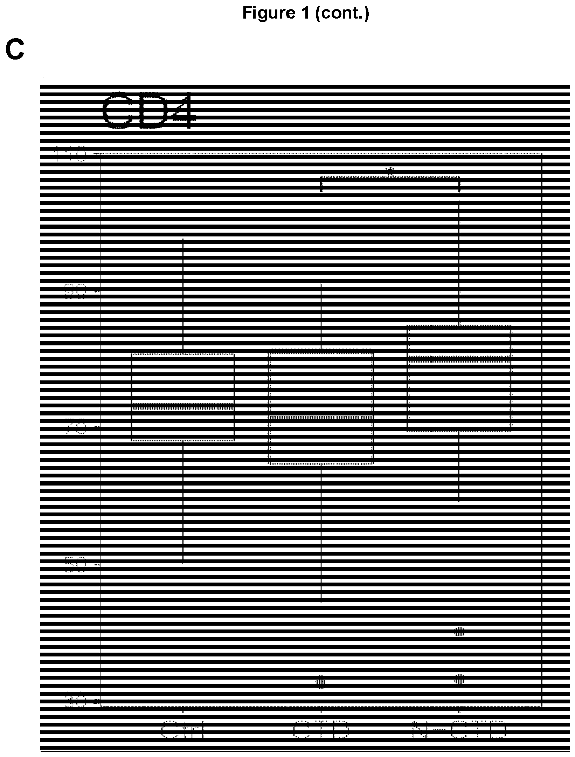

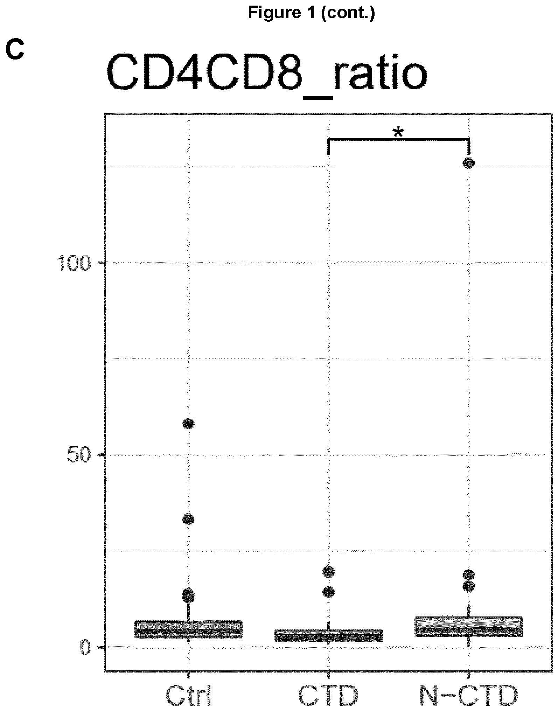

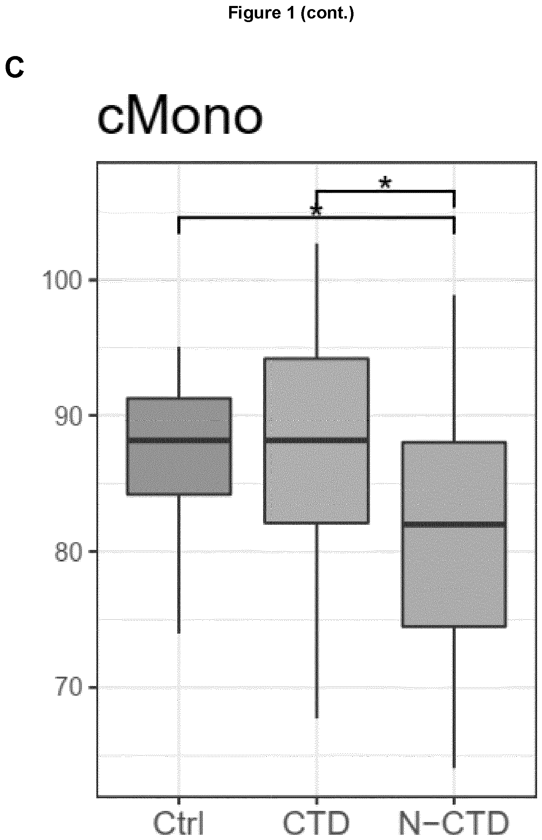

**Fig. 1****: Elevated blood CD4 T cells, CD4CD8 ratio and classical monocytes in N-CTD patients compared to CTD patients. (A)** Scheme illustrating the study design: Cerebrospinal fluid (CSF) and blood from 38 somatoform control patients (Ctrl), 28 connective tissue disease without neurological manifestation (CTD) and 38 connective tissue disease with neurological manifestation (N-CTD) was analyzed by flow cytometry using a predefined antibody panel (Methods) and additional standard CSF analysis was performed as part of the diagnostic workup. Data were queried and analyzed retrospectively. **(B)** Heatmap of the group means of the blood parameters. The group mean of each blood parameter was determined, then normalized and clustered hierarchically (see Methods). **(C)** Blood parameters that showed significant alterations in any comparison are visualized in boxplots categorized by disease group. Immune cell frequencies are shown as percentages of their parent gate (Methods). The boxes display the lower quartile, median, and upper quartile and the whisker includes 1.5 times the interquartile range. The statistical significance was calculated with the Kruskal-Wallis test with post-hoc Dunn's test. The p-values were adjusted with the Benjamini-Hochberg's method. The statistical significance was determined with the Mann Whitney U test. * p < 0.05, ** p < 0.01 *** p < 0.001.

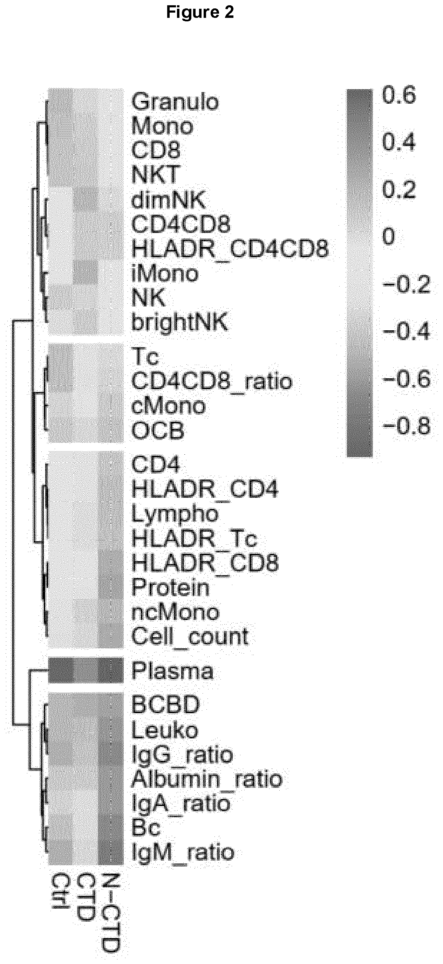

**Fig. 2****: No significant differences in the parameters of the CSF of N-CTD vs. CTD patients.** Heatmap of the group means of the CSF parameters. The group mean of each CSF parameter was calculated, normalized and clustered hierarchically (see Methods).

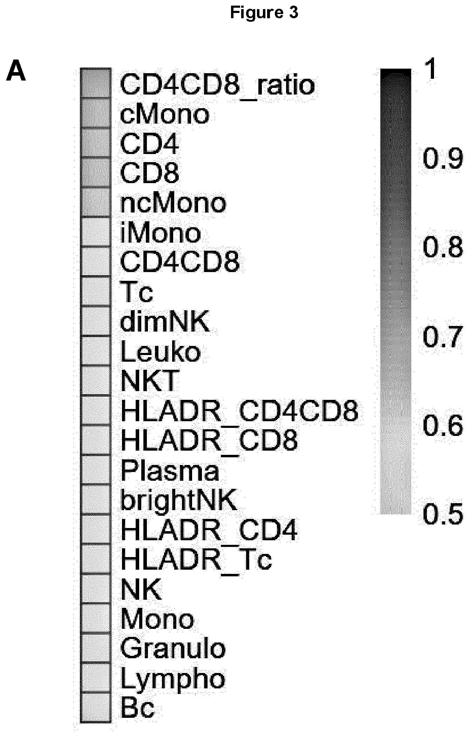

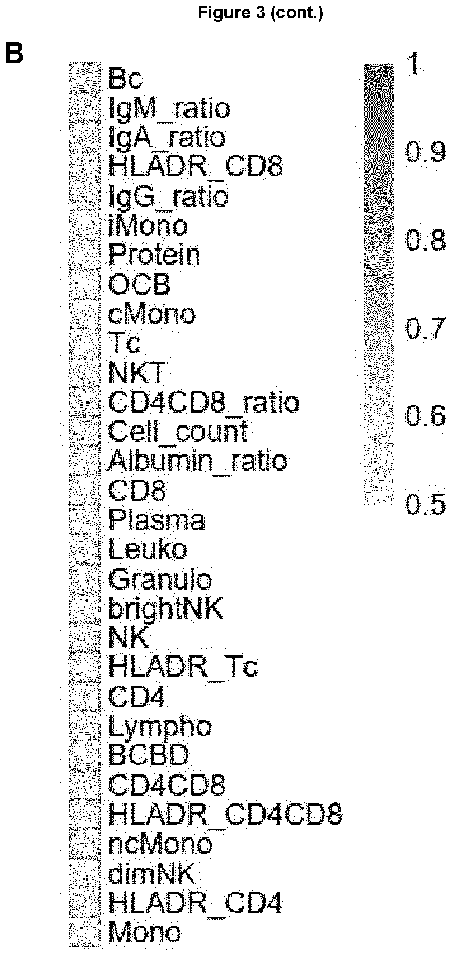

**Fig. 3****: Blood CD4 T cells, a CD4CD8 ratio and classical monocytes can distinguish N-CTD from CTD. (A** and **B)** The area under the curve (AUC) values of the ROC analysis of N-CTD vs. CTD in the blood **(A)** and in the CSF **(B)** are visualized in a heatmap sorted by AUC value. Possible AUC values range from 0.5 (uninformative) to 1 (perfect) and measure the discriminatory ability (combined specificity and sensitivity) of the parameter.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments described throughout the specification should not be construed to limit the present invention to only the explicitly described embodiments. This description should be understood to support and encompass embodiments which combine the explicitly described embodiments with any number of the disclosed and/or preferred elements. Furthermore, any permutations and combinations of all elements described herein should be considered disclosed by the description of the present application unless the context indicates otherwise.

Throughout this specification and the claims which follow, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps although in some embodiments such other member, integer or step or group of members, integers or steps may be excluded, i.e. the subject-matter consists in the inclusion of a stated member, integer or step or group of members, integers or steps. When used herein the term "comprising" can be substituted with the term "containing" or "including" or sometimes when used herein with the term "having". When used herein "consisting of" excludes any element, step, or ingredient not specified.

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein.

All methods described herein can be performed in any suitable order unless otherwise indicated herein or otherwise clearly contradicted by context. The use of any and all examples, or exemplary language (e.g., "such as"), provided herein is intended merely to better illustrate the invention and does not pose a limitation on the scope of the invention otherwise claimed. No language in the specification should be construed as indicating any non-claimed element essential to the practice of the invention.

Unless otherwise indicated, the term "at least" preceding a series of elements is to be understood to refer to every element in the series. The term "at least one" refers to one or more such as one, two, three, four, five, six, seven, eight, nine, ten and more. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

When used herein "consisting of' excludes any element, step, or ingredient not specified in the claim element. When used herein, "consisting essentially of" does not exclude materials or steps that do not materially affect the basic and novel characteristics of the claim.

The term "including" means "including but not limited to". "including" and "including but not limited to" are used interchangeably.

The term "about" means plus or minus 20%, preferably plus or minus 10%, more preferably plus or minus 5%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

Several documents are cited throughout the text of this specification. Each of the documents cited herein (including all patents, patent applications, scientific publications, manufacturer's specifications, instructions, etc.), whether *supra* or *infra,* are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

The content of all documents and patent documents cited herein is incorporated by reference in their entirety.

A better understanding of the present invention and of its advantages will be gained from the examples, offered for illustrative purposes only. The examples are not intended to limit the scope of the present invention in any way.

### Method of the present invention

As used herein, the method of the present invention is applied to diagnose a subject with connective tissue disease with neurological manifestation (N-CTD). Such N-CTD relates *inter alia* to a CTD (a rheumatic / rheumatological disease), wherein connecting structures holding the tissue in a body together are negatively affected and which can be classified according to the ACR/EULAR criteria for example into the subgroups of SLE, GPA, or SS as known to a person skilled, but distinguishes from CTD by further including neurological signs / symptoms. Thus, the neurological manifestation may refer to a neurological sign / symptom attributed to the primary disease CTD comprising *inter alia* SLE, GPA and SS. It may refer to one or more of the neurological signs / symptoms such as, but not limited to, epileptic seizures, polyneuropathy symptoms, inflammatory CNS disease symptoms (such as tissue inflammation), cognitive deficits/dysfunctions, headaches, stroke, psychosis, aseptic meningitis. After having diagnosed a subject with N-CTD with the method of the invention, it is clear that such subject suffers from N-CTD, for example any one of the CTDs as listed above such as SLE, GPA or SS plus any one of the particular neurological manifestations.

The term "diagnose / diagnosing" as used herein and throughout the entire description refers to confirming a diagnosis that a subject which already suffers from rheumatic symptoms as defined elsewhere herein suffers indeed from N-CTD and not from CTD after having applied the method of the present invention to said subject. In this regard, a diagnosis is more concrete and reliable than a stratification of a subject, since it is not associated with the risk of suffering from such disease in the near future, but a concrete detection or determination of such disease.

The term "to have / having N-CTD" can be used interchangeably with the term "to suffer from / suffering from N-CTD". In general, when a subject suffers from a disease, said subject shows specific symptoms of such disease, whereas when a subject has a disease, said subject does not always have to show certain symptoms of the disease, but still is diagnosed with said disease. This does not apply to N-CTD. The subject being diagnosed with N-CTD suffers from N-CTD or has N-CTD, meaning showing neurological manifestations as defined herein and showing symptoms of CTD including *inter alia* SLE, SS or GPA, but which can only be considered as N-CTD with certainty based on the particular requirements being fulfilled by the method of the invention.

The term "subject" as used herein and throughout the entire description, also addressed as an individual, refers to a human or non-human animal, generally a mammal. A subject may be a mammalian species such as a rabbit, a mouse, a rat, a Guinea pig, a hamster, a dog, a cat, a pig, a cow, a goat, a sheep, a horse, a monkey, an ape or a human. Preferably, the subject being used in the present invention is a human. More preferably, said subject is an adult. Preferably, said adult is older than 18 years. More preferably, said adult is about 30 to 76 years, about 40 to 57 years, about 45 to 51 years old. Such subject may also be considered as "patient" when referring to a human, preferably to an adult.

First, as step a) of the method of the present invention, when a subject as defined herein is being examined and which suffers from rheumatic symptoms, the following parameters (specific cell levels) need to be determined in said obtained test PB sample of said subject: i) a level of CD4⁺ T cells; ii) a level of CD8⁺ T cells; and iii) a level of CD14⁺CD16⁻ monocytes. The term "marker" can be used interchangeably with the term "parameter".

The subject suffering from rheumatic / rheumatological symptoms which is examined with the method of the invention shows such specific symptoms as defined below and is then diagnosed with CTD based on the general diagnosing test(s) the skilled person is familiar with before applying the method of the present invention. To determine whether such diagnosed CTD subject - before examining such subject with the method of the invention - may refer to one of the particular subgroups of SLE, GPA or SS, different ACR/EULAR criteria may be applied, which distinguish from one another for the particular subgroups such as for SLE (see 2019 ACR/EULAR criteria as can be seen in Aringer et al. 2019, Arthritis Rheumatol 2019;71:1400-12)), for SS (see 2016 ACR/EULAR criteria as can be seen in Shiboski et al. 2016; Arthritis Rheumatol 2017;69:35-45) and for GPA (see 2022 ACR/EULAR criteria as can be seen in Robson et al. 2022, Arthritis & Rheumatology 2022;74:393-9). CTD (comprising *inter alia* SLE, SS and GPA) as defined elsewhere herein may thus refer to a rheumatic / rheumatological disease. Thus, a subject suffering from rheumatic / rheumatological symptoms, which is then examined with the method of the invention, is diagnosed beforehand with CTD based on the rheumatic symptoms the subject already suffers from applying general diagnosing test(s). The method of the present invention then helps to clarify whether the subject suffering from rheumatic / rheumatological symptoms / being diagnosed with CTD suffers from N-CTD.

In a further embodiment, the subject already suffering from rheumatic symptoms as defined elsewhere herein, which is then examined with the method of the invention can even be suspected to suffer from N-CTD. The term "suspected to suffer" refers to assuming that said subject being examined by using the method of the present invention might suffer from N-CTD based on the first impression the subject already suffering from rheumatic symptoms gives when explaining his/her symptoms and/or based on the general diagnosing test(s) available in the prior art (e.g., MRI), which has/have already been applied to said subject for the diagnosis before the method of the present invention has been applied.

The "rheumatic / rheumatological symptoms" the subject suffers from and which is then diagnosed with CTD before being examined with the method of the present invention may refer to symptoms associated with CTD. Such rheumatic / rheumatological symptoms may refer to at least any one of, but are not limited to, aches, pains, swelling of any one of the joints, muscles, bones, tendons and ligaments, involvement of inner organs (such as heart, kidney, brain, liver, lung, skin and eye and the like), abnormalities of the blood (such as anemia, leukopenia, thrombocytopenia); stiffness of the spine or limited range of motion / movement due to tightening and hardening for example of the skin, immobility, feelings of tiredness or fatigue, fever, weight loss or shiny skin pitting or separation of the nail from the nail bed.

The term "detect" or "detecting" can be used interchangeably with the term "determine" or "determining" as used herein. The term "detect" or "detecting" as well as the term "determine" or "determining" when used herein in combination with the words "level" or "amount", are understood to generally refer to a quantitative or a qualitative level. For example, when used in the context of detecting the level of a certain cell population, such as CD4⁺ T cells, "detect", "detecting", "determine" or "determining" are understood to generally refer to a quantitative level. Accordingly, methods according to the invention that include a quantification of CD3, CD4, CD8, CD14, CD16 and CD45, means i.e. the level of CD3expressing, CD4expressing, CD8expressing, CD14expressing, CD16expressing, and CD45expressing cells. The invention involves detection / determination of the "level", i.e. (relative) amount of CD4⁺ T cells. The level of specific cells may be expressed by the (relative) amount of specific cells being detected. It can also be expressed by the strength of a signal measured in the method of detecting the level of specific cells when immunofluorescence may be used, which may be combined with flow cytometry. The abovementioned can be applied to each cell level (CD8⁺ T cells as well as CD14⁺CD16⁻ monocytes) being determined by the method of the present invention.

Further, after the determination of said particular cell levels in said PB sample from a subject suffering from rheumatic symptoms, for the diagnosis of said subject with N-CTD, as step b) the ratio of the determined level of CD4⁺ T cells of step ai) to the determined level of CD8⁺ T cells of step aii) is determined.

Determining the ratio of the level of CD4⁺ T cells to another level of CD8⁺ T cells as defined above means comparing the level of such specific CD4⁺ T cells to the level of CD8⁺ T cells, or putting said levels of cells into relation to one another. Thus, determining the ratio of the level of CD4⁺ T cells in relation to the level of CD8⁺ T cells may also be comprised herein.

Thus, the term "in relation to" or just "to" as used herein in this context, e.g. "the level of A" (in relation) to "the level of B", generally means comparing "A" to "B". Preferably, this comparison is carried out by dividing A by B, thereby obtaining a ratio value. Here, the relation of "A" to "B" can be expressed as the quotient of "A" divided by "B", again thereby obtaining a ratio value. As an illustrative example, "the level of CD4⁺ T cells" (in relation) to the "level of CD8⁺ T cells" may be expressed by the quotient when dividing "the level of CD4⁺ T cells" by the "level of CD8⁺ T cells". For example, the level of CD4⁺ T cells in relation to the level of CD8⁺ T cells determined in or from a sample of a subject may be expressed in terms of a decimal value or a percentage, i.e. the quotient of dividing the level of CD4⁺ T cells by the level of CD8⁺ T cells. Therefore, determining the ratio of "A" (e.g., the determined level of CD4⁺ T cells) to / in relation to "B" (the determined level of CD8⁺ T cells) means calculating the specific ratios by dividing "A" (e.g., the determined level of CD4⁺ T cells) by the "B" (the determined level of CD8+ T cells).

Next, such step b) as defined above follows as step c) of the method of the present invention the diagnosing step of said subject whether indeed suffering from N-CTD, if certain requirements concerning the particular parameters (determined cell levels of step a) of the method of the invention) as defined below are fulfilled. In particular, the ratio of the level of CD4⁺ T cells to the level of CD8⁺ T cells as well as the level of CD4⁺ T cells need to be increased relative to the corresponding ratios and levels in control PB samples which have been obtained from a cohort of subjects which suffer from CTD. Additionally, the level of CD14⁺CD16⁻ monocytes needs to be decreased relative to the corresponding levels in control PB samples which have been obtained from the same cohort of subjects which suffer from CTD. Then, if the following requirements of step c) are fulfilled (combination of i) to iii) of step c) of the method of the invention), it is indicative that said subject already suffering from rheumatic symptoms and being diagnosed with CTD now also suffers from N-CTD. Thus, said subject can be diagnosed with N-CTD.

If the abovementioned parameters are determined in said obtained samples from said subject being examined (see step a) of the method of the invention), but the requirements for said parameters in accordance with step c) of the method are not fulfilled, the subject most likely does not suffer from N-CTD, thereby said subject cannot be diagnosed with N-CTD. Thus, even if all cell levels as parameters are determined in a PB sample being obtained from said subject already suffering from rheumatic symptoms, but if for example one or more of the requirements (e.g. that the level of CD4⁺ T cells is not increased relative to corresponding levels of CD4⁺ T cells in said control PB samples obtained from said subjects suffering from CTD) is not fulfilled, it is an indication that said subject already suffering from rheumatic symptoms as defined elsewhere herein does not suffer from N-CTD, but suffers from CTD. The method of the present invention thus makes it possible for a subject to be diagnosed with N-CTD if all of the parameters are being determined in said PB sample being obtained from said subject in step a) and if said requirements listed above under step c) of the method of the present invention are fulfilled.

According to the present invention, it may also be comprised that a subject being diagnosed with CTD beforehand, but not being diagnosed as defined herein with N-CTD based on the general stratification test(s) available in the prior art (e.g., imaging test such as X-rays and MRI), which also may have been applied to said subject before, may then be diagnosed with N-CTD by applying the method of the present invention. Thus, the method of the present invention may also diagnose a specific patient group, which has not been diagnosed with N-CTD or which failed to be diagnosed with N-CTD by applying classical stratification procedures known to a person skilled in the art.

When a subject as defined herein is being examined and then diagnosed with N-CTD as mentioned above, a test PB sample is being obtained as known to the person skilled in the art from said subject as defined elsewhere herein. Such sample being obtained from said subject as defined herein is thus examined for said particular parameters of i) to iii) of step a) of the method of the present invention.

The PB sample as used in the method of the present invention is, essentially consists of, or includes PB from such subject suffering from rheumatic symptoms which is then diagnosed with CTD. The term "essentially consists of" is understood to allow the presence of additional components in said PB sample or a composition that do not affect the properties of the sample. Examples of additional components may include, but are not limited to certain types of media, or any type of buffer.

The sample used in the method of the present invention is a PB sample dependent on the fact that each parameter is determined in step a) of the method of the present invention as defined elsewhere herein, since such parameters have not been significant in CSF samples. PB is the flowing, circulating blood of the body. It is composed of erythrocytes, leukocytes and thrombocytes. These blood cells are suspended in blood plasma, through which the blood cells are circulated through the body. On the other hand, CSF which is not used as a sample for the method of the invention is a clear, colorless body fluid found in the brain and spinal cord. It acts as a buffer for the brain, providing basic mechanical and immunological protection to the brain and serving a vital function in cerebral autoregulation of cerebral blood flow. CSF is derived from blood plasma and is largely similar to it. However, CSF is nearly protein-free compared with plasma and has some different electrolyte levels. In general, CSF is substantially free of red blood cells (erythrocytes), and at most contains a few white blood cells (leukocytes).

In the present invention said PB sample may refer to a "test" PB sample. This means that said sample obtained from said subject suffering from rheumatic symptoms according to the present invention is analyzed / examined by determining said specific cell levels as defined by step a) and then determining the particular ratio of CD4⁺ to CD8⁺ T cells as defined by step b) of the method of the present invention, wherein if the requirements of step c) of the method are fulfilled, it is indicative whether said subject suffers from N-CTD. The control PB samples as comparison for whether said particular cell levels / ratio in the test sample as defined in step c) of the method of the present invention are either increased or decreased are taken from a patient cohort which have been diagnosed with CTD. Thus, this patient cohort may also be considered as not being healthy, since they suffer from / have CTD. Again, in this context, the definition concerning the term "to have / having N-CTD" may be applied *mutatis mutandis* herein. Thus, the subject suffering from / having CTD, shows any symptoms associated with CTD including *inter alia* SLE, SS or GPA as known to a person skilled in the art and which have been defined above and is diagnosed with CTD based on general diagnosing test(s) also known to a person skilled in the art.

The methods of the invention may include providing a sample from the subject or obtaining said sample from the subject. The PB sample may be obtained by venous puncture following typical procedures known to a person skilled in the art and then collected in particular tubes for said further determination of said particular cell levels (parameters).

The sample may have been taken at any desired point in time before carrying out the method of the invention. Generally, a time interval between taking the sample and carrying out the method of the invention is selected to allow analysis of viable cells. Cell viability can readily be determined using standard methods known in the art, e.g. neutral red uptake (NRU) or water soluble tetrazolium (WST-1). It is within the skilled artisan's experience to determine a respective time interval during which cells in a sample can be expected to remain viable.

It is envisaged by the invention that the sample may have been taken on the same or on the previous day, such as about 72 hours, about 48 hours, about 42 hours, about 36 hours, about 30 hours, about 28 hours, about 24 hours, about 18 hours, about 15 hours, about 12 hours, about 10 hours, about 8 hours, about 6 hours, about 4 hours, about 2 hours or less before the method of the invention is being carried out. Preferably, it is envisaged by the invention that the sample is taken about 2 hours before the method of the invention is carried out. In other words, said sample preferably needs to be processed within about 2 hours after having obtained said sample. When the sample may be fixed and then analyzed / examined as defined elsewhere herein, it may also be processed within about 72 hours (about 3 days).

The invention also contemplates that the sample from the subject may be a frozen sample. Generally, the sample may be frozen within 0 to about 72 or 0 to about 48, and/or at the above exemplified time points, such as about 72 hours, about 48 hours, about 36 hours or less, preferably about 2 hours, after the sample has been obtained from the subject. A frozen sample may be formed by freezing an obtained sample after adding a cryoprotective agent such as DMSO, glycerol and/or hydroxyethyl starch. As an illustrative example DMSO may be used in a final concentration in the range from about 2% to about 10 %, such as about 2%, about 5% or about 10% DMSO. Typically, the sample is then frozen at a controlled rate to a temperature less than -50°C, whereafter the sample may for instance be stored, including long-term storage, at a temperature below -130°C such as -160°C, e.g. in liquid nitrogen for extended periods of time.

The term "relative to" or just "to" as used within the present invention, e.g. "the level of A" (relative) to "the level of B", generally means comparing "A" to "B". Thus, in the context of the present invention for example a level of CD4⁺ T cells being determined in a test PB sample being obtained from a subject to be examined is then compared to the levels of CD4⁺ T cells in control PB samples taken from a cohort of subjects which have been diagnosed with suffering from CTD, so that the levels of CD4⁺ T cells in the control PB samples can be considered as being corresponding to the level of CD4⁺ T cells in the test PB sample obtained from said subject. The same applies *mutatis mutandis* to the other cell levels / ratio as mentioned in the context of the method of the present invention. Preferably, this comparison is carried out by comparing the parameters, whereas p-values are preferably calculated by the Mann-Whitney U test (see **Figure 1c**). The term "in comparison to" can be used interchangeably with the term "relative to" or just "to".

Additionally or alternatively, Receiver Operating Characteristic (ROS) may be applied as statistical tool to determine a well responding subpopulation (a control patient suffering from / being diagnosed with CTD) from a poorly responding subpopulation (a test patient being diagnosed with N-CTD) (see **Figure 3**). From the frequency of test results (cell levels / ratio according to the method of the invention, see step a)) among such patients (control (control patient suffering from / being diagnosed with CTD) vs. test (a test patient being diagnosed with N-CTD)) based on gold standard, one can derive the probability of a positive test result for patients with disease (N-CTD) (i.e. the conditional probability of correctly identifying the diseased subjects by test-sensitivity or true positive rate (TPR)) and the probability of negative test results for patients without disease (i.e. the conditional probability of correctly identifying non-diseased subjects by test -specificity or true negative rate (TNR)). A false positive in this case occurs when a patient tests positive (poor responder or diagnosed with N-CTD), but actually is a good responder and does not suffer from N-CTD (false positive rate, FPR). A false negative, on the other hand, occurs when the patient tests negative (good responder or not being diagnosed with N-CTD), when it actually suffers from it (false negative rate, FNR).

To draw a ROC curve, the TPR and the FPR are determined as the decision reference / threshold is varied continuously. Since TPR is equivalent with sensitivity and FPR is equal to 1 - specificity, the ROC graph is sometimes called the sensitivity vs (1 - specificity) plot. A perfect test will have an area under the ROC curve (AUC) of 1.0 meaning that the prediction is always correct (TPR = 100%, FPR = 0%); a random test will have an area of 0.5 (TPR = 0%, FPR = 100%).

The term "increased" or "decreased" as used herein and throughout the entire description, relates to an increased level of cells or a decreased level of cells concerning the quantity of the cells in view of their cell number, such term as known to the skilled person. It also refers to an increased ratio of such cell levels in view of their cell number. Thus, any elevation or reduction of the cell number and/or ratio can be considered as a relevant increase or decrease. According to the present invention, any recognizable alteration in form of a higher or a lower cell number in relation to corresponding levels of cells in said control samples also concerning the quantity of the cells in view of their cell number may be considered as an increase or decrease. The increase or decrease of a cell number may be determined using flow cytometry as defined elsewhere herein. As an illustrative example, an increase or decrease may be in the range of at least about 0.4-fold, about 0.5-fold, about 0.6-fold, about 0.7-fold, 0.8-fold, 0.9-fold, 1-fold, about 1.1-fold, about 1.2-fold, about 1.3-fold, about 1.4-fold, about 1.5-fold, about 1.6-fold, about 1.7-fold, about 1.8-fold, about 1.9-fold, about 2-fold, or even about 3-fold.

It is further encompassed by the present invention that said level of CD4⁺ T cells, the level of CD8⁺ cells, and the level of CD14⁺CD16⁻ monocytes are detected / determined using flow cytometry. Flow cytometry-based analysis is typically combined with optical detection to identify and classify cells. This allows speed, sensitivity/specificity, and a non-invasive nature of the technique. Typically, fluorescent markers are used, which are compounds that bind to specific structures or molecules on the surface or within target cells. Such fluorescent markers are introduced into the mixture of cells, whereafter the mixture is rinsed to remove excess fluorescent markers. It is envisaged that flow cytometry may be combined with immunofluorescence. It is thus contemplated by the invention that the level of each specific cells defined above may be determined by detecting the specific surface molecules on the surface of each cells by using flow cytometry being combined with immunofluorescence. Therefore, in the present invention CD3, CD4, CD8, CD14, CD16, and/or CD45 may be detected on the surface of the specific cells in the samples using a flow cytometry based analysis.

Generally, the invention envisages that the detection of the biomarkers CD3, CD4, CD8, CD14, CD16, and/or CD45 can be carried out in a single step or can be carried out in more than one step, e.g. two steps, three steps or four steps. In preferred embodiments, the detection is carried out in a single step. A preferred method for the detection of said specific cell levels as defined above is flow cytometry such as FACS. The skilled person in the art knows that if detection of the biomarkers is to be carried out in more than one step, more than one sequences of steps can be selected. It is understood that the skilled person is able to recognize suitable sequences of steps for identifying the cell populations of interest. Also, if more than one step is carried out, the steps may partly involve enriching and/or isolating subpopulations. Again, the skilled person is able to recognize suitable sequences of steps and can judge whether those steps would reasonably involve an enrichment and/or isolation step for identifying the cell populations of interest.

Flow cytometry is a technique enabling counting, examining, and sorting microscopic particles such as biological cells suspended in a stream of fluid. It allows a simultaneous multiparametric analysis of the physical and chemical characteristics of single cells flowing through an optical detection device. An illustrative example of a well-established flow cytometry based analysis in the art is FACS. FACS allows sorting a heterogeneous mixture of cells into a plurality of containers, one cell at a time, based upon the specific light scattering and fluorescent characteristics of each cell. Thereby, FACS allows the sorting of subpopulations of cells of interest and their further use in *in vitro* and *in vivo* assays. FACS is often used in combination with monoclonal immunoglobulins as a reagent to detect cells as having a particular antigen, indicative of an expressed protein.

This technique allows the concurrent fast, objective and quantitative recording of fluorescent signals from individual cells and the physical separation of respective cells according to particular interest. Fluorescent signals used in flow cytometry, for instance when quantifying and/or sorting cells by any marker present on or in the cell, are typically fluorescently-tagged antibody preparations or fluorescently-tagged ligands for binding to antibodies or other antigen-, epitope- or ligand-specific agent, such as with biotin/avidin binding systems or fluorescently-labeled and optionally addressable beads (e.g. LUMINEX^{®} microspheres). Depending of the equipment used, any desired detectable marker or combination of detectable markers can be detected by the optics of a flow cytometer. Current three-laser "multidimensional", flow cytometry devices enable up to 23 simultaneous single-cell measurements, such as two light scatter detectors and 21 fluorescence plus forward detectors allowing for example the detection of fluorescent surface/intracellular markers. Current three-laser, "multidimensional", FACS machines, if used as an illustrative example of flow cytometry, enable up to 14 simultaneous single-cell measurements, such as two light scatter detectors and 12 fluorescence plus forward detectors allowing for example the detection of fluorescent surface/intracellular markers. As an illustrative example, the three lasers of a flow cytometry device may be a krypton laser operating at 407 nm, an argon laser operating at 488 nm, and a dye laser operating at 595 nm. There are also devices with seven lasers and about 70 channels i.e. so-called spectral flow-cytometer, whereas about 40 channels may be realistic to use, which fall under the term "multidimensional" flow cytometers which may be used by the present invention.

The flow cytometry technique has been used extensively in relation to antigens expressed on the surface of cells, including cells that remain alive during, and after, flow cytometry. Similarly, the method has been used with intracellular reporter gene systems based on the expression of a detectable labeled gene product by the cell. Accordingly, the technique not only allows detecting the presence of CD3, CD4, CD8, CD14, CD16, and/or CD45 on the cell surface, but also detecting the presence of RNA or DNA within the cell, for example RNA encoding CD3, CD4, CD8, CD14, CD16, and/or CD45. Therefore, flow cytometry can also be used to determine the amount of nucleic acid formation from the genes, which encode CD3, CD4, CD8, CD14, CD16, and/or CD45 in cells of the sample from the subject.

In preferred embodiments when diagnosing a subject with N-CTD, flow cytometry technique is used for detecting the level of CD4⁺ T cells, preferably by detecting each specific cell surface marker / molecule such as CD3, CD4, and CD45 on the cell surface of said cells, which demonstrates a certain strength of a signal being measured.

In detail, determining the level of CD4⁺ T cells within the method of the present invention comprises measuring one or more of the markers selected from the group consisting of CD3, CD4, and CD45. In the context of the present invention, distinct markers represent essential markers. Accordingly, CD45 can be considered as a pan leukocyte marker, which should be included everywhere. Further, CD45 can be considered as essential marker for the distinct subsets of cells, such as for CD4⁺ T cells: *inter alia* CD3 and CD4; for CD8⁺ T cells: *inter alia* CD3 and CD8; for monocytes: *inter alia* CD14 and CD16. The present invention thus contemplates that the presence of the CD45 on the surface of a cell may be used to identify the cell as a leukocyte. CD45 (Cluster of Differentiation 45 or leukocyte common antigen) protein is a member of the protein tyrosine phosphatase (PTP) family and is expressed by all leukocytes. PTPs are known to be signaling molecules that regulate a variety of cellular processes including cell growth, differentiation, mitotic cycle, and oncogenic transformation. This PTP contains an extracellular domain, a single transmembrane segment and two tandem intracytoplasmic catalytic domains, and thus belongs to receptor type PTP. CD45 is a type I transmembrane protein that is in various forms present on all differentiated hematopoietic cells, except erythrocytes and plasma cells that assist in the activation of those cells (a form of co-stimulation).

Additionally, the invention contemplates that the presence of CD3 (Cluster of Differentiation 3) and/or CD4 (Cluster of Differentiation 4) on the surface of a cell may be used to identify the cell as CD4+ T cell. In general, T cells are known to the skilled artisan as lymphocytes, i.e. nucleated blood cells that are also called white blood cells. T cells mature in the thymus and can be distinguished from other lymphocytes in that they have the T cell receptor on their cell surface. The main known role of the T cell is recognition of antigens bound to major histocompatibility complex (MHC) molecules. The T cell receptor (TCR) is a heterodimer, which consists of a 34 kD α-chain, linked by a disulphide bond to a 34 kD β-chain in about 95 % of T cells. Both chains span the plasma membrane and have accordingly an extracellular portion, each of which includes a variable region, termed Vα and Vβ, respectively. About 5 % of T cells have a T cell receptor that consists of a γ- and a δ-chain instead of an α - and a β-chain, which likewise have extracellular variable regions. T cell receptors can, like immunoglobulins, recognize a very large number of different epitopes. As the T cell receptor has variable regions it may, nevertheless, be advantageous to use another cell surface protein to identify a T cell. An example of suitable protein in this regard is a T cell co-receptor. An illustrative example of a co-receptor of the T cell receptor is the protein complex CD3 (Cluster of Differentiation 3). CD3 has four chains, which are in mammals one CD3γ chain (e.g. human CD3γ of the UniProt accession number P09693, version 147 of 7 January 2015 or corresponding mRNA of the GenBank Accession number X04145, version X04145.1 GI:37021 of 18 April 2005), one CD3δ chain (e.g. human CD3δ of the UniProt accession number P04234, version 157 of 7 January 2015 or corresponding mRNA of the GenBank Accession number BC039035, version BC039035.1 GI:25058311 of 19 June 2006), and two CD3ε chains (e.g. human CD3ε of the UniProt accession number P07766, version 167 of 7 January 2015 or corresponding mRNA of the GenBank Accession number X03884, version X03884.1 GI:37039 of 07 April 1994). These chains associate with a molecule known as the T-cell receptor and at least one T-cell surface glycoprotein CD3 zeta chain also known as T-cell receptor T3 zeta chain or CD247 (Cluster of Differentiation 247) (e.g. human CD3 zeta chain of the UniProt accession number P20963, version 165 of 7 January 2015 or corresponding mRNA of the GenBank Accession number J04132, version J04132.1 GI:623041 of 12 January 1995). The complex of TCR, CD247 and CD3 can generate an activation signal in T lymphocytes. The TCR, ζ-chain(s), and CD3 molecule together define the TCR complex. In practicing the methods according to the invention identifying the presence of CD3 on a particular cell or plurality of cells is often a convenient way of identifying T cells. Therefore, the terms "CD3+ T cell" and "T cell" are used interchangeable herein to address a T cell and to distinguish a T cell from other cell types.

According to the invention, identifying CD3+ T cells in the sample serves in distinguishing CD3+ T cells from other cells such as CD3- cells or non-T cells (such as NK cells and/or B cells). In the methods according to the invention, the level of T cells in the sample that is CD4 positive (CD4+) are detected. Identifying CD4+ T cells typically serves in distinguishing CD4+ T cells from other cells such as CD4- T cells. CD4+ T cells in addition to CD3 have the CD4 (Cluster of Differentiation 4) protein on their surface, a glycoprotein consisting of four extracellular immunoglobulin domains, termed D1 to D4, and a small cytoplasmic region (e.g. human CD4 of the UniProt accession number P01730, version 180 of 7 January 2015 or corresponding mRNA of the GenBank Accession number M12807, version M12807.1 GI:179141 of 27 April 1993). CD4+ T cells can be classified into a variety of cell populations with different functions and should thus not be taken to define a unitary set of cells. Typical examples of a CD4+ T cell are naive CD4+ T-cells (naive), "central" memory CD4+ T-cells (TCM), or "effector" memory CD4+ T cells (TEM).

Additionally, determining the level of CD8⁺ T cells with the method of the present invention comprises measuring of one or more of the markers selected from the group consisting of CD3, CD8, and CD45. Identifying CD8+ T cells typically serves in distinguishing CD8+ T cells from other cells such as CD8- T cells. CD8+ T cells in addition to CD3 have the CD8 (Cluster of Differentiation 8) protein on their surface, a transmembrane glycoprotein consisting of a pair of CD8 chains, most commonly composed of a CD8- α and CD8- β chain having a molecular weight of about 34 kDa. The CD8 co-receptor is predominantly expressed on the surface of cytotoxic T cells. Like the TCR, CD8 interacts with the MHC molecule, but is specific for the Class I MHC molecule (in particular it interacts with α₃ portion of the Class I MHC molecule).

Further, determining the level of CD14⁺CD16⁻ monocytes comprises measuring of one or more of the markers selected from the group consisting of CD14, CD16, and CD45. Also the invention contemplates that the presence of CD14 (Cluster of Differentiation 14) and/or CD16 (Cluster of Differentiation 16) on the surface of a cell may be used to identify the cell as a monocyte. CD16 may also be expressed not only on monocytes, but also on NK cells. Thus, another surface molecule may be determined to detect the level of monocytes, such as CD14. Monocytes represent a heterogeneous population of primary immune effector cells. They are the largest type of leukocyte and can differentiate into macrophages and myeloid lineage dendritic cells. There are three different subsets being distinguished based on their expression of CD14 and the low-affinity CD16 (FcγRIII): CD14++CD16- classical monocytes, which are characterized by high level expression of the CD14 cell surface receptor. CD14++CD16+ intermediate monocytes, which are characterized by high level expression of CD14 and low-level expression of CD16 and CD14+CD16++ non-classical monocytes, which are characterized by low level expression of CD14 and high co-expression of the CD16 receptor. According to the present invention, the level of monocytes as being determined in step a) of the method of the present invention preferably refers to CD14+CD16- monocytes which refer to classical monocytes as defined above.

The term "measuring a surface marker / molecule" refers to measuring the signal of a detectable marker, which is attached to a binding partner that recognizes the specific surface marker / molecule expressed by the specific cell when using flow cytometry. Said signal may then be converted by the process called gating, which is known to a person skilled in the art, which then demonstrates the level (or relative amount) of cells being detected in said sample. The gating comprises among other factors measuring of distinct cell populations using forward scatter (FSC) and side scatter (SSC). Measuring FSC and SSC in combination allows to some extend the differentiation of a cell population within heterogenous cell populations. The determination of FSC allows the discrimination of cells by size. The determination of SCS allows the determination of internal complexity of the cells (such as granularity). For example, intracellular granules and the nucleus increase SSC.

The measurement used is generally selected to be of a sensitivity of detection that allows detection of at least any one of CD3, CD4, CD8, CD14, CD16, or CD45 expressing cells in the range of a selected threshold value, in particular of a sensitivity of detection that allows determining whether at least any one of CD3, CD4, CD8, CD14, CD16, or CD45 expressing cells are above the threshold.

It is understood that if detection is carried out using flow cytometry, a binding partner of at least any one of CD3, CD4, CD8, CD14, CD16, and/or CD45 may be used, preferably linked to excitable fluorescent dyes or proteins. Such a binding partner of CD3, CD4, CD8, CD14, CD16, and/or CD45 has a detectable affinity and specificity for CD3, CD4, CD8, CD14, CD16, and/or CD45. Typically, binding is considered specific when the binding affinity is higher than 10⁻⁶ M. A binding partner of CD3, CD4, CD8, CD14, CD16, and/or CD45 has in some embodiments an affinity of about 10⁻⁸ M or higher, or of about 10⁻⁹ M or higher. As indicated above, in some embodiments for example CD4⁺ T cells in the sample are identified by the presence of the CD4 protein on their surface; or CD4⁺ T cells may be enriched or isolated via the presence of the CD4 protein on their surface. Identification of CD4⁺ T cells may be carried out using spectroscopic, photochemical, photometric, fluorometric, radiological, enzymatic or thermodynamic means. Identification and enrichment or isolation of CD4⁺ T cells may likewise be carried out by using a suitable binding partner of CD4+ T cells. Accordingly, the abovementioned applies *mutatis mutandis* to identifying and enriching or isolating all kinds of immune cells, classical and non-classical monocytes, CD8+ T cells, by using a suitable binding partner of their specific surface molecules and optionally a further suitable binding partner of a surface protein characteristic for said cells, e.g. when T cells may be identified or isolated in a similar manner using additionally a suitable binding partner of a surface protein characteristic for T cells as known in the art, for example the T cell receptor.

Immunofluorescence being used in flow cytometry is generally achieved using a binding partner, which is linked to, or includes, a fluorophore as a detectable marker. Typically, a binding partner of CD4 f.e. may be used in combination with a detectable marker or the binding partner is functionally linked to a detectable marker. Likewise, a binding partner of CD3, CD8, CD14, CD16, and/or CD45 may be used in combination with a detectable marker or the binding partner is functionally linked to a detectable marker.

In some embodiments suitable binding partners of at least any one of CD3, CD4, CD8, CD14, CD16, or CD45 are combined to identify cells expressing a combination of biomarkers selected from CD3, CD4, CD8, CD14, CD16, and CD45. In an illustrative example, suitable binding partners of at least any one of CD3, CD4 or CD45 are combined to identify CD4⁺ T cells. In another illustrative example, suitable binding partners of at least any one of CD3, CD8 or CD45 are combined to identify CD8⁺ T cells. In a further illustrative example, suitable binding partners of at least any one of CD14, CD16 or CD45 are combined to identify monocytes. The abovementioned binding partners may be functionally linked to a detectable marker.

In a preferred embodiment of the present invention, a suitable binding partner of CD3 and a suitable binding partner of CD4 are combined to identify CD4⁺ T cells. In a further preferred embodiment of the invention, a suitable binding partner of CD3 and a suitable binding partner of CD8 are combined to identify CD8⁺ T cells. In a further preferred embodiment of the invention, a suitable binding partner of CD14 and a suitable binding partner of CD16 are combined to identify monocytes.

A respective binding partner of at least any one of CD3, CD4, CD8, CD14, CD16, or CD45 and optionally as well as a binding partner for another selected cell-characteristic protein, may be an immunoglobulin or a fragment thereof or a proteinaceous binding molecule with immunoglobulin-like functions. An example of a proteinaceous binding molecule with immunoglobulin-like functions is a mutein based on a polypeptide of the lipocalin family (WO 03/029462, Beste et al., Proc Nat. Acad Sci 1999; 96:1898-1903). Lipocalins, such as the bilin binding protein, the human neutrophil gelatinase-associated lipocalin, human Apolipoprotein D or glycodelin, possess natural ligand-binding sites that can be modified so that they bind to selected small protein regions known as haptens. Examples of other proteinaceous binding molecules are the so-called glubodies (see e.g. international patent application WO 96/23879 or Napolitano et al., Chemistry & Biology 1996; 3(5):359-367), proteins based on the ankyrin scaffold (Mosavi et al., Protein Science 2004; 13(6):1435-1448) or crystalline scaffold (e.g. internation patent application WO 01/04144), the proteins described in Skerra, J. Mol. Recognit. 2000; 13:167-187, AdNectins, tetranectins and avimers. Avimers contain so called A-domains that occur as strings of multiple domains in several cell surface receptors (Silverman et al., Nature Biotechnology 2005; 23:1556-1561). Adnectins, derived from a domain of human fibronectin, contain three loops that can be engineered for immunoglobulin-like binding to targets (Gill & Damle, Current Opinion in Biotechnology 2006; 17:653-658). Tetranectins, derived from the respective human homotrimeric protein, likewise contain loop regions in a C-type lectin domain that can be engineered for desired binding.

An immunoglobulin or a proteinaceous binding molecule with immunoglobulin-like functions may be PEGylated or hyperglycosylated if desired. In some embodiments a proteinaceous binding molecule with immunoglobulin-like functions is a fusion protein of one of the exemplary proteinaceous binding molecules above and an albumin-binding domain, for instance an albumin-binding domain of streptococcal protein G. In some embodiments, a proteinaceous binding molecule with immunoglobulin-like functions is a fusion protein of an immunoglobulin fragment, such as a single-chain diabody, and an immunoglobulin binding domain, for instance a bacterial immunoglobulin binding domain.

An immunoglobulin used in the method of the invention may be an antibody. Thus, an immunoglobulin fragment thereof may be an antibody fragment having antibody activity. It generally contains an antigen binding or variable region. Examples of (recombinant) antibody fragments are immunoglobulin fragments such as Fab fragments, Fab' fragments, Fv fragments, single-chain Fv fragments (scFv), diabodies or domain antibodies (Holt, L.J., et al., Trends Biotechnol. (2003), 21, 11, 484-490). A suitable antibody may in some embodiments also be a multispecific antibody that includes several immunoglobulin fragments. An immunoglobulin may be monoclonal or polyclonal. The term "polyclonal" refers to immunoglobulins that are heterogenous populations of immunoglobulin molecules derived from the sera of animals immunized with an antigen or an antigenic functional derivative thereof. For the production of polyclonal immunoglobulins, one or more of various host animals may be immunized by injection with the antigen. Various adjuvants may be used to increase the immunological response, depending on the host species. "Monoclonal immunoglobulins", also called "monoclonal antibodies", are substantially homogenous populations of immunoglobulins to a particular antigen. They may be obtained by any technique which provides for the production of immunoglobulin molecules by continuous cell lines in culture. Monoclonal immunoglobulins may be obtained by methods well known to those skilled in the art (see for example, Köhler et al., Nature (1975) 256, 495-497, and U.S. Patent No. 4,376,110). An immunoglobulin or immunoglobulin fragment with specific binding affinity only for said particular cell surface markers as defined elsewhere herein can be isolated, enriched, or purified from a prokaryotic or eukaryotic organism. Routine methods known to those skilled in the art enable production of both immunoglobulins or immunoglobulin fragments and proteinaceous binding molecules with immunoglobulin-like functions, in both prokaryotic and eukaryotic organisms. In more detail, an immunoglobulin may be isolated by comparing its binding affinity to a protein of interest, e.g. CD4, with its binding affinity to other polypeptides. Humanized forms of the antibodies of the present invention may be generated using one of the procedures known in the art such as chimerization or CDR grafting. In general, techniques for preparing monoclonal antibodies and hybridomas are well known in the art. Any animal such as a goat, a mouse or a rabbit that is known to produce antibodies can be immunized with the selected polypeptide, e.g. CD4. Methods for immunization are well known in the art. Such methods include subcutaneous or intraperitoneal injection of the polypeptide. One skilled in the art will recognize that the amount of polypeptide used for immunization and the immunization regimen will vary based on the animal which is immunized, including the species of mammal immunized, its immune status and the body weight of the mammal, as well as the antigenicity of the polypeptide and the site of injection.

As indicated above, a detectable marker may be coupled to a binding partner of CD3, CD4, CD8, CD14, CD16, and/or CD45 as the case may be, or a molecule that forms a complex with the binding partner of CD3, CD4, CD8, CD14, CD16, and/or CD45. A respective detectable marker, which may be coupled to a binding partner of CD3, CD4, CD8, CD14, CD16, and/or CD45 or a molecule that forms a complex therewith, may be a chromophore, an excitable fluorescent dye, a radioactive amino acid, a fluorescent protein, an enzyme, a hapten, a digoxigenin, a biotin, a metal complex, or metal and colloidal gold. Examples of a suitable fluorescent dye include, but are not limited to, Krome Orange (KrO), fluorescein (FITC), fluorescein isothiocyanate, 5,6-carboxymethyl fluorescein, Cascade Blue^{®}, Oregon Green^{®}, Texas red, nitrobenz-2-oxa-1,3-diazol-4-yl, coumarin, dansyl chloride, rhodamine, amino-methyl coumarin, DAPI, Eosin, Erythrosin, BODIPY^{®}, pyrene, lissamine, xanthene, acridine, a fluorescent brightener (PB), an oxazine, phycoerythrin, a Cy dye such as Cy3, Cy3.5, Cy5, Cy5PE, Cy5.5, Cy7, Cy7PE or Cy7APC, an Alexa dye such as Alexa 647, Alexa 750 or Alexa 700, and NBD (Naphthol basic dye). Examples of a suitable fluorescent protein include, but are not limited to, EGFP, emerald, EYFP, a phycobiliprotein such as phycoerythrin (PE) or allophycocyanin (APC), Monomeric Red Fluorescent Protein (mRFP), mOrange, mPlum and mCherry. In some embodiments a reversibly photoswitchable fluorescent protein such as Dronpa, bsDronpa and Padron may be employed (Andresen, M., et al., Nature Biotechnology (2008) 26, 9, 1035). Regarding suitable enzymes, alkaline phosphatase, soybean peroxidase, or horseradish peroxidase may serve as a few illustrative examples. In a further embodiment, tandem conjugates such as PE-Cy5.5 or PE-Cy7 may also be used. In some embodiments a method of detection may include electrophoresis, HPLC, flow cytometry, fluorescence correlation spectroscopy or a modified form of these techniques. Some or all of these steps may be part of an automated separation/detection system. In some embodiments, a detectable marker being coupled to a binding partner as defined herein may include a "fluorescently labelled binding partner".

In a preferred example, KrO may be used as a detectable marker (fluorescent dye) of the binding partner for CD45 as defined herein. PE-Cy5.5 may be used as a detectable marker (fluorescent dye) of the binding partner for CD3 as defined herein. APC may be used as a detectable marker (fluorescent protein) of the binding partner for CD4 as defined herein. PB may be used as a detectable marker (fluorescent dye) of the binding partner for CD8 as defined herein. FITC may be used as a detectable marker (fluorescent dye) of the binding partner for CD14 as defined herein. Alexa 750 may be used as a detectable marker (fluorescent dye) of the binding partner for CD16 as defined herein.

### Computer-implemented features

The subject-matter of the defined method steps of the present invention may also fully be carried out by computer program instructions running on means which, in the context of the invention, provide generic data processing functions. Such means may, for example, be embedded in a personal computer, smartphone, printer. A computer-implemented invention may therefore be one which involves the use of a computer, computer network or other programmable apparatus, where one or more features are realized wholly or partly by means of a computer program. Thus, all of the abovementioned definitions concerning the method of the invention may be applied *mutatis mutandis* also to the computer-implemented features now defined herein.

The present invention comprises a data processing system comprising a processor configured to perform a method comprising the steps of obtaining i) the detected level of CD4⁺ T cells; ii) the detected level of CD8⁺ cells; and iii) the detected level of CD14⁺CD16⁻ monocytes, from a test peripheral blood (PB) sample obtained from a subject suffering from rheumatic symptoms and determining the ratio of the detected level of CD4⁺ T cells to the detected level of CD8⁺ T cells and then diagnosing said subject as suffering from N-CTD, if all the requirements as mentioned elsewhere herein are fulfilled, since the combination of such requirements is indicative for whether said subject already suffering from rheumatic symptoms suffers from N-CTD.

According to the present invention, said specific cell levels as defined above may be detected in a test PB sample obtained from a subject suffering from rheumatic symptoms which is then also diagnosed with CTD according to the present invention using flow cytometry, in particular using a flow cytometry device. The detected level of specific cells being expressed in terms a decimal value or a percentage as described elsewhere herein may be entered into said data processing system comprising a processor which is configured to perform the abovementioned steps. In some embodiments, the step of obtaining said detected cell levels as defined above may be an optional step. As used herein, by using the term "obtaining / obtain the detected level of specific cells" in this context means that the data for the detected level (in percentage) of the defined cells being detected in a flow cytometry device as defined elsewhere herein is entered into the data processing system by any way known to a person skilled in the art. The step of comparing the detected cell levels / ratio to the corresponding levels / ratios in the control samples for said particular diagnosis is performed as defined elsewhere herein within the processor.

A more meaningful characterization of modern digital data processing systems is the functional classification as either computational or input/output ("I/O") oriented. As the classifying labels imply, computational oriented data processing systems are designed primarily for performing long, complicated calculations. I/O oriented data processing systems are designed to handle large quantities of digital data, thereby requiring extensive I/O operations. Both data processing systems may be comprised by the present invention. The data processing system of the present invention may also be utilized as a peripheral subsystem of a larger computational computer. The structural design of a data processing system may necessarily directly be related to the functional use to which the data processing system is put. A data processing system of the present invention may refer to a programmable or programmed device / apparatus, where one or more features are realized wholly or partly by means of a computer program. A data processing system may include, but are not limited to, a computer, a smartphone, a tablet, or a chip.

As used herein, the term "processor" may refer to one functional element being a physical unit of a data processing system, which is configured by a computer program as defined elsewhere herein to perform the specified steps mentioned above.

The present invention further comprises the interaction between the data processing steps and other technical means such as a flow cytometry device. Thus, the present invention comprises a flow cytometry device capable of detecting i) the level of CD4⁺ T cells; ii) the level of CD8⁺ cells; and iii) the level of CD14⁺CD16⁻ monocytes in a test PB sample obtained from a subject suffering from rheumatic symptoms, comprising the data processing system as defined above.

Said specific cell levels may be detected by using a flow cytometry device, preferably using flow cytometry analysis being combined with immunofluorescence as described elsewhere herein. As an illustrative example, said flow cytometry device as defined above may be a FACS. In some embodiments said flow cytometry such as for example FACS may be connected to a computer, a tablet, a smartphone or any other technical device / apparatus being used as a further means for performing said flow cytometry analysis.

The present invention also envisages a computer program comprising instructions to cause the data processing system as defined above or the flow cytometry device as defined above to execute the steps of obtaining i) the detected level of CD4⁺ T cells; ii) the detected level of CD8⁺ cells; and iii) the detected level of CD14⁺CD16⁻ monocytes, from a test PB sample obtained from a subject suffering from rheumatic symptoms and determining the ratio of the detected level of CD4⁺ T cells to the detected level of CD8⁺ T cells and then diagnosing said subject as suffering from N-CTD, if the following are fulfilled:
i) the determined ratio of the detected level of CD4⁺ T cells to the detected level of CD8⁺ T cells is increased relative to corresponding ratios of the level of CD4⁺ T cells to the level of CD8⁺ T cells in control PB samples obtained from subjects suffering from CTD;
ii) the detected level of CD4⁺ T cells is increased relative to corresponding levels of CD4⁺ T cells in said control PB samples obtained from said subjects suffering from CTD; and
iii) the detected level of CD14⁺CD16⁻ monocytes is decreased relative to corresponding levels of CD14⁺CD16⁻ monocytes in said control PB samples obtained from said subjects suffering from CTD, wherein the combination of i) - iii) is indicative for whether said subject already suffering from rheumatic symptoms suffers from N-CTD.

Again, in some embodiments, the step of obtaining said detected cell levels as defined above may be an optional step. Everything being defined above for said data processing system may apply *mutatis mutandis* to the corresponding computer program as defined herein.

According to the present invention a computer program as mentioned above may refer to a program listing written in a programming language to implement an algorithm, and to binary code loaded in a computer-based apparatus encompassing the accompanying documentation. A computer program as defined above may include, but is not limited to any software or any downloadable internet link known to a person skilled in the art comprising instructions to cause the data processing system as defined elsewhere herein to execute the defined steps.

Also comprised by the present invention is a computer-readable medium having stored thereon the computer program as defined above. As used herein, a computer-readable medium having stored thereon the computer program may include, but is not limited to, a USB stick, a disc, DVD, CD, CD-ROM.

### EXAMPLES OF THE INVENTION

The following Examples illustrate the invention, but are not to be construed as limiting the scope of the invention.

### Material and Methods

### Patient characteristics.

The inventors retrospectively screened patients with the ICD-10 diagnosis M32.-(systemic lupus erythematosus, short: SLE), M35.0 (sjögren syndrome, short: SS), and M31.3 (granulomatosis with polyangiitis, short: GPA), and F45.- (somatoform disorder) (control) who were admitted to the University Hospital Münster between 2011 and 2020 and received a diagnostic lumbar puncture (LP) for obtaining CSF samples and received a diagnostic venous puncture (VB) for obtaining peripheral blood (PB) samples including flow cytometry. CTD patients were selected according to the ACR/EULAR criteria. SLE patients fulfilled the 2019 ACR/EULAR criteria for SLE (Aringer et al. 2019, Arthritis Rheumatol 2019;71:1400-12), SS patients the 2016 ACR/EULAR criteria for SS (Shiboski et al. 2016; Arthritis Rheumatol 2017;69:35-45), and GPA patients the 2022 ACR/EULAR criteria for GPA (Robson et al. 2022, Arthritis & Rheumatology 2022;74:393-9)*.*

### Flow cytometry and basic CSF analysis.

LPs were performed under sterile conditions and CSF was promptly analyzed. CSF was centrifuged at 300 g for 15 min, the supernatant was removed and CSF cells were stained with different antibodies (e.g. Heming et al. 2019, Frontiers in Immunology, 10). In short: flow cytometry was performed on a Navios flow cytometer (Beckman Coulter). Cells were incubated in VersaLyse buffer and stained with anti-human antibodies (Beckman Coulter, clone names in brackets): CD3 (UCHT1); CD4 (13B8.2); CD8 (B9.11); CD14 (RMO52); CD16 (3G8); CD19 (J3-119); CD45 (J.33); CD56 (C218); CD138 (B-A38); HLA-DR (Immu-357). CSF cells were counted with a Fuchs-Rosenthal chamber. Protein concentration, IgA, IgG, and IgM levels were studied by nephelometry. A Reiber scheme was created and evaluated for the presence of a blood-CSF-barrier-disruption (BCBD). Oligoclonal bands (OCBs) were detected via isoelectric focusing followed by silver nitrate staining.

### Flow cytometry and basic PB analysis.

Blood (PB) was collected using standard procedures such as diagnostic venous puncture (VB) and was promptly analyzed. Blood cells were incubated in VersaLyse buffer and blood cells were stained using the following anti-human antibodies (Beckman Coulter, clone names in brackets): CD3 (UCHT1); CD4 (13B8.2); CD8 (B9.11); CD14 (RMO52); CD16 (3G8); CD19 (J3-119); CD45 (J.33); CD56 (C218); CD138 (B-A38); HLA-DR (Immu-357). Collected blood was layered on top of LymphoprepTM (Stemcell) and gradient centrifugation was performed in accordance with manufacturer's instructions. After centrifugation, PBMCs enriched in the interface were taken off and washed in 10 ml of media. PBMC were then used for staining.

### Statistics.

The computational analysis was carried out with R 4.1.1. The Mann-Whitney U test was used when comparing two groups and the Kruskal Wallis test with the Dunn test as a post hoc test when comparing multiple groups. Multiple testing correction was performed with the Benjamini-Hochberg's procedure. A p-value of < 0.05 was considered statistically significant.

The inventors adjusted for the therapy with the *datawizard* R package. Briefly, a linear model was fitted for each variable with the therapy status as a predictor. The residuals were computed and used for further analysis. The boxplots, the variable importance plot, and the ROC curves were generated with the *ggplot2 R* package. Significance in the boxplots was annotated with the *ggsignif* package. The correlation plot was produced with the R package *corrplot* based on the Spearman coefficient. The data were clustered hierarchically with complete linkage and the Euclidean distance measure. The heatmaps were created with the R package *pheatmap.* In a first step, Yeo-Johnson transformation was performed followed by centering and scaling with the *recipes R* package. Next, the mean of each parameter for each cohort was calculated and visualized in a heatmap. Rows were clustered hierarchically with complete linkage and Euclidean distance measure. The inventors performed receiver operating characteristic (ROC) analysis with the R package pROC. A ROC analysis permits a systematic evaluation of the sensitivity and specificity of a test represented by the area under the curve (AUC) values, which range from 0.5 (uninformative) to 1 (perfect). The machine learning algorithms were evaluated with the *tidymodels R* package. First, the data were split into training (75%) and test (25%) data stratified according to the outcome. The data were then preprocessed with a Yeo-Johnson transformation, filtering out near-zero variance, and centered and scaled. Lasso regression was performed with the *glmnet* package. The model was trained with repeated 10-fold cross-validation with 100 repeats to take the small number of samples into account. The best model was chosen based on the highest AUROC. The resulting variable importance scores were determined with the *R* package *vip.*

### Results

### Example 1: Patient cohort and interdependency of individual flow cytometry parameters.

The inventors retrospectively analyzed flow cytometry data of paired blood and CSF samples and CSF 'basic parameters' (see Methods) of 28 CTD and 38 N-CTD patients, who had been admitted to the study over 10 years and received a lumbar and a venous puncture as part of the diagnostic workup (see **Figure 1A**). Notably, all CSF samples collected during regular working hours were analyzed using a standardized flow cytometry panel covering most leukocyte lineages (see Methods). A detailed clinical characterization of the CTDs, including organ manifestations, antibody status, ACR/EULAR scores, and neurological signs and symptoms of each patient was extracted from the electronic health records. The largest proportion of patients was diagnosed with SLE and a smaller proportion with GPA or SS (see **Table 1**). Consequently, the age and sex ratios were comparable across all groups (see **Table 1**). The inventors then first tested for the interdependence of the available blood and CSF parameters and identified several modules of correlating parameters (data not shown).

**Table 1: Characteristics of study patients.**

| | n | median age | % female | GPA | SLE | SS |
|---|---|---|---|---|---|---|
| Ctrl | 38 | 46.4 | 76.3 | 0 | 0 | 0 |
| CTD | 28 | 50.1 | 67.9 | 6 | 17 | 5 |
| N-CTD | 38 | 46.6 | 76.3 | 4 | 29 | 5 |

### Example 2: Specific blood parameters distinguish N-CTD from CTD.

The Examiner next aimed to understand how connective tissue diseases affected blood and CSF and first focused on blood parameters. Blood plasma cells were significantly elevated in both CTD and N-CTD compared to Control (Ctrl) patients (see **Figures 1B** and **C**). The inventors next sought to identify blood parameters that distinguished N-CTD from CTD patients. It was found that CD4⁺ T cells and the CD4⁺/CD8⁺ ratio increased in N-CTD compared to CTD, while classical monocytes were decreased in N-CTD (see **Figures 1B** and **C**). Neurological involvement of systemic autoimmunity is thus associated with specific alterations of innate and adaptive immune cells that can already be detected in the blood. This indicates a diagnostic potential.

The inventors next investigated whether analysis of the CSF, which is in constant contact with the brain borders, could shed light on 'neurology-specific' mechanisms in CTDs. As expected for autoimmune diseases affecting the brain, BCBD, cell counts, plasma cells, and B cells were all significantly increased in the CSF compared to somatoform controls (see **Figure 2A**)**.** This indicates intrathecal B cell expansion. However, no single CSF parameter differed significantly between N-CTD and CTD. In stark contrast to the primary brain autoimmune disease MS, laboratory surrogates distinguishing N-CTD from CTD are thus more readily detectable in the blood (see **Figure 1C****)** than in CSF (see **Figure 2A**)**.**

### Example 3: Receiver Operator Curve Analysis proves high relevance of blood parameters to distinguish N-CTD vs. CTD.

Aiming to facilitate diagnostic decisions, the inventors next systematically quantified the discriminatory power of individual blood and CSF parameters with a receiver operator curve (ROC) analysis. The resulting area under the curve (AUC) classifies a biomarker between perfect (AUC 1.0) and useless (AUC 0.5) with AUC 0.7-0.8 usually considered as good. The inventors focussed on clinically relevant comparisons between N-CTD vs. CTD. In blood, the CD4⁺/CD8⁺ T cell ratio (AUC 0.68) was the best parameter to distinguish N-CTD from CTD patients (see **Figure 3A**)**.** Interestingly, CSF parameters performed worse in differentiating N-CTD from CTD (B cells: AUC 0.63) (see **Figure 3B**).

### ITEMS

1. A method of diagnosing a subject with connective tissue disease with neurological manifestation (N-CTD), comprising
   a) determining
      i) a level of CD4⁺ T cells;
      ii) a level of CD8⁺ T cells; and
      iii) a level of CD14⁺CD16⁻ monocytes
      in a test peripheral blood (PB) sample obtained from a subject suffering from rheumatic symptoms, and
   b) determining the ratio of the determined level of CD4⁺ T cells of step ai) to the determined level of CD8⁺ T cells of step aii);
   c) diagnosing said subject as suffering from N-CTD, if the following are fulfilled:
      i) the ratio of the level of CD4⁺ T cells to the level of CD8⁺ T cells of step b) is increased relative to corresponding ratios of the level of CD4⁺ T cells to the level of CD8⁺ T cells in control PB samples obtained from subjects suffering from CTD;
      ii) the level of CD4⁺ T cells is increased relative to corresponding levels of CD4⁺ T cells in said control PB samples obtained from said subjects suffering from CTD; and
      iii) the level of CD14⁺CD16⁻ monocytes is decreased relative to corresponding levels of CD14⁺CD16⁻ monocytes in said control PB samples obtained from said subjects suffering from CTD,
      wherein the combination of i) - iii) of step c) is indicative for whether said subject already suffering from rheumatic symptoms suffers from N-CTD.
2. The method of item 1, wherein the level of CD4⁺ T cells, the level of CD8⁺ cells, and the level of CD14⁺CD16⁻ monocytes are detected using flow cytometry.
3. The method of item 1 or 2, wherein determining the level of CD4⁺ T cells comprises measuring of one or more of the markers selected from the group consisting of CD3, CD4, and CD45.
4. The method of any one of items 1-3, wherein determining the level of CD8⁺ T cells comprises measuring of one or more of the markers selected from the group consisting of CD3, CD8, and CD45.
5. The method of any one of items 1-4, wherein determining the level of CD14⁺CD16⁻ monocytes comprises measuring of one or more of the markers selected from the group consisting of CD14, CD16, and CD45.
6. The method of any one of items 1-5, wherein a binding partner for at least any one of CD3, CD4, CD8, CD14, CD16, or CD45 is used.
7. The method of item 6, wherein the binding partner is an immunoglobulin or a fragment thereof, or a proteinaceous binding molecule with immunoglobulin-like functions.
8. The method of any one of items 1-7, wherein said subject is a human.
9. The method of any one of items 1-8, wherein said subject is an adult.
10. The method of any one of items 1-9, wherein said subject is suspected to suffer from N-CTD.
11. A data processing system comprising a processor configured to perform a method comprising the steps of
   a) obtaining
      i) the detected level of CD4⁺ T cells;
      ii) the detected level of CD8⁺ cells; and
      iii) the detected level of CD14⁺CD16⁻ monocytes,
      from a test peripheral blood (PB) sample obtained from a subject suffering from rheumatic symptoms and determining the ratio of the detected level of CD4⁺ T cells of step ai) to the detected level of CD8⁺ T cells of step aii);
   b) diagnosing said subject as suffering from N-CTD, if the following are fulfilled:
      i) the determined ratio of the detected level of CD4⁺ T cells to the detected level of CD8⁺ T cells is increased relative to corresponding ratios of the level of CD4⁺ T cells to the level of CD8⁺ T cells in control PB samples obtained from subjects suffering from CTD;
      ii) the detected level of CD4⁺ T cells is increased relative to corresponding levels of CD4⁺ T cells in said control PB samples obtained from said subjects suffering from CTD; and
      iii) the detected level of CD14⁺CD16⁻ monocytes is decreased relative to corresponding levels of CD14⁺CD16⁻ monocytes in said control PB samples obtained from said subjects suffering from CTD,
      wherein the combination of i) - iii) of step b) is indicative for whether said subject already suffering from rheumatic symptoms suffers from N-CTD.
12. A flow cytometry device capable of detecting
   i) the level of CD4⁺ T cells;
   ii) the level of CD8⁺ cells; and
   iii) the level of CD14⁺CD16⁻ monocytes;
   in a test PB sample obtained from a subject suffering from rheumatic symptoms, comprising the data processing system of item 11.
13. A computer program comprising instructions to cause the data processing system of item 11 or the flow cytometry device of item 12 to execute the steps of
   a) obtaining
      i) the detected level of CD4⁺ T cells;
      ii) the detected level of CD8⁺ cells; and
      iii) the detected level of CD14⁺CD16⁻ monocytes,
      from a test PB sample obtained from a subject suffering from rheumatic symptoms and determining the ratio of the detected level of CD4⁺ T cells of step ai) to the detected level of CD8⁺ T cells of step aii);
   b) diagnosing said subject as suffering from N-CTD, if the following are fulfilled:
      i) the determined ratio of the detected level of CD4⁺ T cells to the detected level of CD8⁺ T cells is increased relative to corresponding ratios of the level of CD4⁺ T cells to the level of CD8⁺ T cells in control PB samples obtained from subjects suffering from CTD;
      ii) the detected level of CD4⁺ T cells is increased relative to corresponding levels of CD4⁺ T cells in said control PB samples obtained from said subjects suffering from CTD; and
      iii) the detected level of CD14⁺CD16⁻ monocytes is decreased relative to corresponding levels of CD14⁺CD16⁻ monocytes in said control PB samples obtained from said subjects suffering from CTD,
      wherein the combination of i) - iii) of step b) is indicative for whether said subject already suffering from rheumatic symptoms suffers from N-CTD or from CTD.
14. A computer-readable medium having stored thereon the computer program of item 13.

## Claims

1. A method of diagnosing a subject with connective tissue disease with neurological manifestation (N-CTD), comprising
a) determining
i) a level of CD4⁺ T cells;
ii) a level of CD8⁺ T cells; and
iii) a level of CD14⁺CD16⁻ monocytes
in a test peripheral blood (PB) sample obtained from a subject suffering from rheumatic symptoms, and
b) determining the ratio of the determined level of CD4⁺ T cells of step ai) to the determined level of CD8⁺ T cells of step aii);
c) diagnosing said subject as suffering from N-CTD, if the following are fulfilled:
i) the ratio of the level of CD4⁺ T cells to the level of CD8⁺ T cells of step b) is increased relative to corresponding ratios of the level of CD4⁺ T cells to the level of CD8⁺ T cells in control PB samples obtained from subjects suffering from CTD;
ii) the level of CD4⁺ T cells is increased relative to corresponding levels of CD4⁺ T cells in said control PB samples obtained from said subjects suffering from CTD; and
iii) the level of CD14⁺CD16⁻ monocytes is decreased relative to corresponding levels of CD14⁺CD16⁻ monocytes in said control PB samples obtained from said subjects suffering from CTD,
wherein the combination of i) - iii) of step c) is indicative for whether said subject already suffering from rheumatic symptoms suffers from N-CTD.

2. The method of claim 1, wherein the level of CD4⁺ T cells, the level of CD8⁺ cells, and the level of CD14⁺CD16⁻ monocytes are detected using flow cytometry.

3. The method of claim 1 or 2, wherein determining the level of CD4⁺ T cells comprises measuring of one or more of the markers selected from the group consisting of CD3, CD4, and CD45.

4. The method of any one of claims 1-3, wherein determining the level of CD8⁺ T cells comprises measuring of one or more of the markers selected from the group consisting of CD3, CD8, and CD45.

5. The method of any one of claims 1-4, wherein determining the level of CD14⁺CD16⁻ monocytes comprises measuring of one or more of the markers selected from the group consisting of CD14, CD16, and CD45.

6. The method of any one of claims 1-5, wherein a binding partner for at least any one of CD3, CD4, CD8, CD14, CD16, or CD45 is used.

7. The method of claim 6, wherein the binding partner is an immunoglobulin or a fragment thereof, or a proteinaceous binding molecule with immunoglobulin-like functions.

8. The method of any one of claims 1-7, wherein said subject is a human.

9. The method of any one of claims 1-8, wherein said subject is an adult.

10. The method of any one of claims 1-9, wherein said subject is suspected to suffer from N-CTD.

11. A data processing system comprising a processor configured to perform a method comprising the steps of
a) obtaining
i) the detected level of CD4⁺ T cells;
ii) the detected level of CD8⁺ cells; and
iii) the detected level of CD14⁺CD16⁻ monocytes,
from a test peripheral blood (PB) sample obtained from a subject suffering from rheumatic symptoms and determining the ratio of the detected level of CD4⁺ T cells of step ai) to the detected level of CD8⁺ T cells of step aii);
b) diagnosing said subject as suffering from N-CTD, if the following are fulfilled:
i) the determined ratio of the detected level of CD4⁺ T cells to the detected level of CD8⁺ T cells is increased relative to corresponding ratios of the level of CD4⁺ T cells to the level of CD8⁺ T cells in control PB samples obtained from subjects suffering from CTD;
ii) the detected level of CD4⁺ T cells is increased relative to corresponding levels of CD4⁺ T cells in said control PB samples obtained from said subjects suffering from CTD; and
iii) the detected level of CD14⁺CD16⁻ monocytes is decreased relative to corresponding levels of CD14⁺CD16⁻ monocytes in said control PB samples obtained from said subjects suffering from CTD,
wherein the combination of i) - iii) of step b) is indicative for whether said subject already suffering from rheumatic symptoms suffers from N-CTD.

12. A flow cytometry device capable of detecting
i) the level of CD4⁺ T cells;
ii) the level of CD8⁺ cells; and
iii) the level of CD14⁺CD16⁻ monocytes;
in a test PB sample obtained from a subject suffering from rheumatic symptoms, comprising the data processing system of claim 11.

13. A computer program comprising instructions to cause the data processing system of claim 11 or the flow cytometry device of claim 12 to execute the steps of
a) obtaining
i) the detected level of CD4⁺ T cells;
ii) the detected level of CD8⁺ cells; and
iii) the detected level of CD14⁺CD16⁻ monocytes,
from a test PB sample obtained from a subject suffering from rheumatic symptoms and determining the ratio of the detected level of CD4⁺ T cells of step ai) to the detected level of CD8⁺ T cells of step aii);
b) diagnosing said subject as suffering from N-CTD, if the following are fulfilled:
i) the determined ratio of the detected level of CD4⁺ T cells to the detected level of CD8⁺ T cells is increased relative to corresponding ratios of the level of CD4⁺ T cells to the level of CD8⁺ T cells in control PB samples obtained from subjects suffering from CTD;
ii) the detected level of CD4⁺ T cells is increased relative to corresponding levels of CD4⁺ T cells in said control PB samples obtained from said subjects suffering from CTD; and
iii) the detected level of CD14⁺CD16⁻ monocytes is decreased relative to corresponding levels of CD14⁺CD16⁻ monocytes in said control PB samples obtained from said subjects suffering from CTD,
wherein the combination of i) - iii) of step b) is indicative for whether said subject already suffering from rheumatic symptoms suffers from N-CTD or from CTD.

14. A computer-readable medium having stored thereon the computer program of claim 13.
